# EUROPÄISCHE PATENTSCHRIFT

(11) **EP 4 132 410 B1**
(45) Veröffentlichungstag und Bekanntmachung des Hinweises auf die Patenterteilung: **16.07.2025**
(21) Anmeldenummer: 21717824.3
(22) Anmeldetag: 07.04.2021
(51) Int. Cl.: B65D 45/22, A61B 50/30

(54) **VERSCHLUSSMECHANISMUS MIT SPERREINRICHTUNG FÜR STERILCONTAINER UND STERILCONTAINER DAMIT**
CLOSURE MECHANISM WITH LOCKING DEVICE FOR STERILE CONTAINERS, AND STERILE CONTAINER COMPRISING SAME
MÉCANISME DE FERMETURE AVEC DISPOSITIF DE VERROUILLAGE POUR RÉCIPIENTS STÉRILES, ET RÉCIPIENT STÉRILE LE COMPRENANT

(30) Priorität: 08.04.2020 DE 102020109765
(43) Veröffentlichungstag der Anmeldung: 15.02.2023
(73) Patentinhaber: Aesculap AG, 78532 Tuttlingen (DE)
(72) Erfinder: BOHNENSTENGEL, Philipp, 78256 Steißlingen (DE); THOMAS, Stefan, 78532 Tuttlingen (DE); ELISCH, Andreas, 78655 Dunningen (DE); HENKE, Matthias, 78048 Villingen-Schwenningen (DE)
(74) Vertreter: Winter, Brandl - Partnerschaft mbB
(86) Internationale Anmeldenummer: PCT/EP2021/059018
(87) Internationale Veröffentlichungsnummer: WO 2021/204847

(56) Entgegenhaltungen:
- WO-A1-2020/011932
- DE-U1- 202013 002 232
- JP-A- 2016 155 557
- US-A1- 2008 000 899
- US-B1- 6 592 000

## Beschreibung

Die vorliegende Erfindung betrifft einen Verschlussmechanismus für einen Sterilcontainer mit einer Containerwanne und einem mittels des Verschlussmechanismus mit der Containerwanne verriegelbaren Containerdeckel, wobei der Verschlussmechanismus ein Riegelelement zum hinterschneidenden Eingriff mit einem eine Hinterschneidung ausbildenden Eingriffselement an der Containerwanne bzw. am Containerdeckel aufweist, wobei das Riegelelement in eine Schließstellung, in der es mit dem Eingriffselement in hinterschneidendem Wirkeingriff steht, und in eine Freigabestellung positionierbar ist, in der das Riegelelement und das Eingriffselement außer Eingriff sind.

Aus dem Stand der Technik sind eingangs beschriebene Sterilcontainer bekannt, die eine mittels eines Containerdeckels hermetisch verschließbare Containerwanne sowie einen Verschlussmechanismus haben, über welchen der Deckel mit der Wanne fluiddicht verriegelbar ist. Dazu ist der sogenannte Bügelverschluss bekannt. Dieser hat einen schwenkbar beispielsweise am Deckel gelagerten Betätigungshebel, an dem ein Bügel oder eine Kralle/Spannpratze angelenkt ist, der/die mit einer seitlich, beispielsweise an der Containerwanne vorragenden Leiste in hinterschneidenden Eingriff kommt und so bei einem Umklappen des Betätigungshebels den Containerdeckel gegen die Containerwanne zieht. Zwischen Deckel und Wanne ist ein Dichtungsring angeordnet, der bei Anziehen des Deckels gegen die Wanne und den Deckel gequetscht wird und so den Kontaktbereich zwischen Wanne und Deckel abdichtet. Dabei können auch geringe Toleranzen ausgeglichen werden.

Beispielsweise beschreibt die WO 2020 / 011 932 A1 einen Verschluss- bzw. Verriegelungsmechanismus für einen Sterilcontainer. Dieser Mechanismus bewirkt ein Anziehen eines Containerdeckels gegen eine Containerwanne. Auch die US 6 592 000 B1 offenbart einen Verschlussmechanismus für einen (Steril-)Container.

Bei Sterilcontainern ist es wichtig, dass sich ihr Innendruck gegenüber dem Umgebungsdruck in einem vorbestimmten Verhältnis befindet. Um dieses zu bewirken, sind Sterilcontainer mit einem Druckausgleichventil bekannt. Um ein Einströmen von Fluid (Luft, Gas) aus der Umgebung in den Containerinnenraum (über eine entkeimende Filtereinrichtung) und/oder ein Ausströmen von Fluid (Luft, Gas) aus dem Containerinnenraum in die Umgebung (unter Umgehung der Filtereinrichtung) zu ermöglichen, ist bekannt, zum Beispiel in dem Containerdeckel eine Ventileinrichtung anzuordnen. Entscheidend für eine korrekte Funktion einer solchen Ventileinrichtung ist eine sichere und fluiddichte Verriegelung des Deckels auf/an der Containerwanne, um ungewollte Leckagen in/aus dem Container an der Kontaktstelle zwischen Wanne und Deckel zu vermeiden.

Eine weitere Möglichkeit, einen Druckausgleich bei solchen Sterilcontainern zu bewirken, ist, einen Verschlussmechanismus derart auszubilden, dass sich der Deckel zum Ausgleich eines zu hohen Innendrucks im Behälter auch im verriegelten Zustand ein wenig von der Containerwanne abheben kann, so dass Fluid aus dem Innenraum ausströmen kann. Dazu ist ein Sterilcontainer mit einem Verschluss-/Verriegelungsmechanismus (im Folgenden Verschlussmechanismus), einer Containerwanne und einem mittels des Verschlussmechanismus mit der Containerwanne verriegelbaren Containerdeckel bekannt. Der Verschlussmechanismus besitzt einen Betätigungshebel, der am Containerdeckel oder an der Containerwanne vorzugsweise schwenkbar gelagert ist, einen Zugbügel / Kralle / Spannpratze (im Folgenden Zugbügel genannt), der / die mit dem Betätigungshebel direkt oder indirekt gekoppelt ist, und ein eine Hinterschneidung ausbildendes Eingriffselement an der Containerwanne oder am Containerdeckel, mit dem der Zugbügel in hinterschneidenden Wirkeingriff bringbar ist, um bei einer Betätigung des Betätigungshebels eine Zugkraft in Richtung der Aufsetzrichtung des Deckels auf den Behälter auf das Eingriffselement auszuüben. Der Zugbügel ist quer, vorzugsweise in einem rechten Winkel zur auszuübenden Zugkraft verschiebbar gelagert. Es ist außerdem ein Vorspannelement zum Beispiel in Form einer Feder vorgesehen, das relativ zum Zugbügel derart in einer Wirkposition angeordnet ist, dass es erst bei einer vorbestimmten Betätigungsstellung des Betätigungshebels und demzufolge einer vorbestimmten/vorbestimmbaren Verschiebeposition des Zugbügels, in der der Zugbügel das Eingriffselement bereits hintergreift, eine Druckkraft auf den Zugbügel in Richtung der auszuübenden Zugkraft, also in die Aufsetzrichtung des Deckels auf dem Behälter, anlegt.

Wichtig bei Verschlussmechanismen und Sterilcontainern ist, dass
- die Containerwanne / das Behälterunterteil und/oder der Containerdeckel / das Behälteroberteil und/oder der Verschlussmechanismus vor einer Beschädigung infolge einer nicht bestimmungsgemäßen / fehlerhaften Betätigung des Verschlussmechanismus zu schützen, insbesondere wenn der Verschlussmechanismus mit einer Hebelfunktion versehen ist, wobei sehr große Kräfte entstehen können,
- an der Stirnseite des Behälterunterteils trotz des Verschlussmechanismus eine möglichst große Fläche zur Kennzeichnung des Behälters / Sterilcontainers frei bleibt,
- ein versehentliches Öffnen der Verschlusseinheit weit möglichst verhindert wird,
- auch größere Fertigungstoleranzen ausgeglichen werden können und
- eine Mindestanzugskraft zwischen Deckel und Wanne gewährleistet ist.

Darüber hinaus wäre es wünschenswert, wenn
- die Zugkraft nicht plötzlich sondern fortlaufend mit fortschreitendem Umlegen des Betätigungshebels zunimmt, insbesondere um Beschädigungen des Verschlussmechanismus und/oder der Dichtung zu vermeiden,
- der Verschlussmechanismus gleichzeitig auch eine Überdruckventilfunktion übernehmen kann,
- der Betätigungshebel an einer gut zugänglichen Stelle, vorzugsweise an der Oberseite des Containerdeckels liegt, um ein seitliches Vorragen auch während des Öffnungs-/Schließvorgangs zu vermeiden und die Stirnseite der Containerwanne nicht abzudecken,
- der Verschlussmechanismus in seiner geschlossenen / verriegelten Endposition möglichst stabil fixiert und vor einem ungewollten / versehentlichen Öffnen gesichert ist und
- der Verschlussmechanismus aus der geschlossenen / verriegelten Endposition bei einer gewollten Betätigung besonders einfach und schnell zu öffnen ist.

Bei bekannten Verschlussmechanismen bzw. Sterilcontainern ist von Nachteil, dass diese nicht alle der vorstehend genannten Eigenschaften bewirken können.

Vor diesem Hintergrund liegt der vorliegenden Erfindung die Aufgabe zugrunde, die genannten Nachteile des Stands der Technik zu verringern, insbesondere einen Verschlussmechanismus zur Verfügung zu stellen, der in seiner geschlossenen Endposition (Schließstellung) möglichst stabil fixiert und vor einem ungewollten / versehentlichen Öffnen gesichert ist, der aus der geschlossenen Endposition / Schließstellung bei einer gewollten Betätigung besonders einfach und schnell zu öffnen ist und bei dem die vorstehend genannten Eigenschaften möglichst alle erreichbar sind.

Der Kerngedanke der vorliegenden Erfindung besteht im Wesentlichen darin, den erfindungsgemäßen Container - Verschlussmechanismus mit einem Spannpratzen- oder Krallen- artigen Schieber/Riegelelement auszurüsten, der beispielsweise auf einem Containerdeckel verschiebbar gelagert ist, wobei dessen Klauenabschnitt (vorzugsweise grundsätzlich, d.h. in jeder Schiebeposition) über den Containerdeckelrand ragt. Zwischen dem Schieber/Riegelelement und dem Containerdeckel ist mindestens ein Druckfederelement angeordnet, welches den Schieber/Riegelelement mit zunehmender Verschiebebewegung aus seiner Freigabeposition (weit außerhalb des Containerdeckelrands) in seine Schließposition (nahe dem Containerdeckelrand) immer stärker vom Containerdeckel (nach oben) wegdrückt, d. h. anhebt.

Des Weiteren ist mindestens eine Rückstellfeder angeordnet, welche den Schieber/Riegelelement in Richtung hin zu seiner Freigabeposition vorspannt.

Schließlich ist der Schieber/Riegelelement oder das (mit dem Schieber für eine gemeinsame Schiebebewegung verbundene) Druckfederelement mit einem (vorzugsweise manuell betätigbaren bzw. freigebbaren) Halteelement/Halteabschnitt versehen/ausgebildet, das mit einem (vorzugsweise manuell betätigbaren bzw. freigebbaren) Sperr-/Eingriffselement am bzw. auf Seiten des Containerdeckels bei/mit Erreichen der Schließposition (unmittelbar und rastend) in Halteingriff bringbar ist, um den Schieber entgegen der Vorspannkraft der Rückstellfeder in Schließposition zu halten, in welcher der Krallenabschnitt einen Containerwannenrand untergreift und den Containerdeckel infolge der Federkraft des mindestens einen Druckfederelements gegen den Containerwannenrand anzieht, so lange, bis das Sperr-/Eingriffselement auf Seiten des Containerdeckels oder das Halteelement/Halteabschnitt auf Seiten des Druckfederelements manuell für ein Freigeben des Schiebers/Riegelelements betätigt wird und der Schieber/Riegelelement aufgrund der Rückstellfeder in seine Freigabeposition zurückschnellt.

In anderen Worten ausgedrückt wird die vorstehende Aufgabe nach der vorliegenden Erfindung gelöst durch einen Verschlussmechanismus nach Anspruch 1, also einen Verschlussmechanismus für einen/eines Sterilcontainer(s) mit einer Containerwanne und einem mittels des Verschlussmechanismus mit der Containerwanne verriegelbaren Containerdeckel, wobei der Verschlussmechanismus ein klauenartiges Riegelelement/Schieber zum hinterschneidenden Eingriff mit einem eine Hinterschneidung ausbildenden Eingriffselement (Containerwannenrand / Randkragen) an der Containerwanne bzw. am Containerdeckel aufweist, wobei das Riegelelement/Schieber in eine Schließstellung, in der es mit dem Eingriffselement in hinterschneidendem Wirkeingriff steht, und in eine Freigabestellung positionierbar ist, in der das Riegelelement und das Eingriffselement außer Eingriff sind, wobei der Verschlussmechanismus ein mit dem Riegelelement/Schieber wirkverbundenes oder wirkverbindbares Sperrelement aufweist, das derart in den Verschlussmechanismus integriert ist, dass es den Verschlussmechanismus bei Erreichen der Schließstellung in dieser Schließstellung sperrt, insbesondere automatisch / ohne gesonderte nutzerzeitige Einwirkung sperrt. Dabei weist das Sperrelement eine Raststruktur, insbesondere in Form einer Schulter oder eines Hakens, auf. Diese Raststruktur gelangt bei die Schließstellung erreichendem oder in der Schließstellung befindlichem Verschlussmechanismus mit einer am Riegelelement als eine Stange angeordnete oder ausgebildete Gegenraststruktur in Eingriff und sperrt derart in einfacher und zuverlässiger Weise den Verschlussmechanismus in der Schließstellung. Die Sperrung bzw. Entsperrung erfolgt bevorzugt in einer Richtung quer zur Bewegungsrichtung des Riegelelements, also insbesondere quer zur Containerdeckelebene oder parallel dazu.

Die Aufgabe wird außerdem gelöst durch einen Sterilcontainer mit einem Containerdeckel und einer Containerwanne und einem erfindungsgemäßen Verschlussmechanismus, insbesondere mit einem Verschlussmechanismus nach einem der angehängten Ansprüche.

Es ist ein besonderer Vorteil der Erfindung, dass der Verschlussmechanismus infolge des eingebauten Sperrelements in der Schließstellung automatisch gesichert werden kann bzw. ist. Dabei kann der Verschlussmechanismus insbesondere derart ausgebildet sein, dass das Sperrelement zwangsläufig bei einem Verbringen des Riegelelements aus der Freigabestellung in die Schließstellung seine Sperrfunktion ausübt, zum Beispiel indem es positionsfest relativ zum Containerdeckel angeordnet ist und mit dem Riegelelement unmittelbar oder mittelbar in Wirkeingriff gelangt oder indem es positionsfest relativ zum Riegelelement angeordnet ist und mit einer positionsfest mittelbar oder unmittelbar am Containerdeckel angeordneten Gegenraststruktur in Wirkeingriff gelangt. Derart kann auf einfache und sichere Weise ein versehentliches oder unbeabsichtigtes Öffnen des Verschlussmechanismus verhindert werden.

Das Riegelelement ist vorzugsweise ein Formteil / Stanzformteil aus Metall oder ein Kunststoffteil. Es kann insbesondere hakenförmig ausgebildet sein, mit einem randseitig über einen Seitenrand des Containerdeckels ragenden Hakenabschnitt zum Hintergreifen des Eingriffselements und einem sich an den Hakenabschnitt anschließenden in die Deckelebene hineinragenden Abschnitt / Schieberabschnitt, der parallel zur Deckelebene positionierbar an dem Containerdeckel geführt / gelagert ist. Das Riegelelement ist vorzugsweise parallel zur Deckelebene verschiebbar, und zwar zwischen der Freigabeposition, in der der Hakenabschnitt und das Eingriffselement nicht miteinander in Eingriff stehen, und der Schließstellung, in der der Hakenabschnitt und das Eingriffselement miteinander in Eingriff stehen, der Verschlussmechanismus gesperrt ist und der Containerdeckel an der Containerwanne gesichert ist. Man kann daher auch sagen, dass das Riegelelement in der Schließstellung durch das Sperrelement gesichert und gesperrt ist.

Vorteilhafte Ausführungsformen der Erfindung sind in den Unteransprüchen beansprucht und werden nachfolgend näher erläutert.

Der Verschlussmechanismus kann nach einer Ausführungsform eine Grundplatte aufweisen, die zur Anordnung / Befestigung des Verschlussmechanismus an dem Containerdeckel ausgebildet und bestimmt ist. Die Grundplatte, die insbesondere als Metallstanzteil / Metallformteil ausgebildet ist, kann eine Lagerstruktur für das Riegelelement aufweisen, vorzugswiese in Form von umgebogenen Randabschnitten oder Kragenabschnitten. In diesen sind vorzugsweise Langlöcher oder -nuten ausgebildet, in denen das Riegelelement oder eine damit verbundene Lagerstruktur aufgenommen und geführt / gelagert sind. Das Riegelelement ist parallel zur Deckelebene linearpositionierbar gelagert. Zusätzlich kann es quer zur Deckelebene schwenkbar sein, so dass ein Anordnen / Ineingriffbringen des Riegelelements mit dem Eingriffselement der Containerwanne erleichtert wird / ist.

Nach einer besonders vorteilhaften Ausführungsform weist der Verschlussmechanismus ein Federelement auf, das ihn in die Freigabestellung vorspannt. Dieses Federelement bewirkt, dass der Verschlussmechanismus bzw. das Riegelelement ohne Sicherung durch das Sperrelement durch die Vorspannung selbsttätig die Freigabestellung einnehmen würde. Erfindungsgemäß verhindert das Sperrelement aber auch bei dieser Ausführungsform ein ungewolltes / versehentliches Übergehen des Verschlussmechanismus aus der Schließstellung in die Freigabestellung. Ein solches Übergehen / eine solche Bewegung ist infolge der Erfindung nur möglich, wenn das Sperrelement zum Beispiel infolge einer nutzerseitigen Betätigung entsperrt wird. Dabei ist es von besonderem Vorteil und anwenderfreundlich, dass der Verschlussmechanismus nach dem Entsperren des Sperrelements infolge der Vorspannung durch das Federelement automatisch und ohne weitere nutzerseitige Einwirkung aus der Schließstellung in die Freigabestellung übergeht. Das Federelement kann insbesondere unmittelbar auf das Riegelelement einwirken, zum Beispiel indem es einerseits am Riegelelement angeordnet / abgestützt ist und andererseits relativ zum Containerdeckel positionsdefiniert / ortsfest angeordnet / gehalten ist.

Nach einer weiteren Ausführungsform der Erfindung kann das Sperrelement zumindest abschnittsweise elastisch ausgebildet und/oder angeordnet sein. Alternativ kann es vollständig elastisch ausgebildet sein. Es kann zum Beispiel als ein Sperr-Federelement oder ein Sperr-Feder-Dämpfer-Element ausgebildet sein, insbesondere durch ein Blattfederelement. Es ist von besonderem Vorteil, wenn das Sperrelement elastisch vorgespannt ausgebildet ist. Insbesondere kann es als ein elastisch vorgespanntes Sperr-Hebelelement (Kipphebel) oder ein elastisch vorgespannter Sperr-Druckknopf ausgebildet sein. Vorzugsweise ist das Sperrelement derart ausgebildet und angeordnet, dass es bei Erreichen der Schließstellung des Verschlussmechanismus / des Riegelelements aufgrund seiner Vorspannung die den Verschlussmechanismus / das Riegelelement in der Schließstellung sperrende Sperrstellung einnimmt. Es ist außerdem von Vorteil, wenn das Sperrelement derart ausgebildet und angeordnet ist, dass es durch eine nutzerseitige Betätigung entgegen seiner Vorspannung aus der Sperrstellung in eine den Verschlussmechanismus / das Riegelelement freigebende Freigabestellung überführbar ist.

Der Verschlussmechanismus nach einer weiteren Ausführungsform der Erfindung kann einen schwenkbar am Containerdeckel bzw. an der Containerwanne gelagerten / lagerbaren Betätigungshebel aufweisen. Dieser kann derart mit dem Riegelelement direkt oder indirekt gekoppelt sein, dass das Riegelelement mittels des Betätigungshebels zumindest in die Schließstellung positionierbar ist und nach einer weiteren Ausführungsform zwischen der Schließstellung und der Sperrstellung positionierbar ist.

Eine weitere Ausführungsform ist dadurch gekennzeichnet, dass das Riegelelement derart ausgebildet und angeordnet ist, dass es im Wirkeingriff mit dem Eingriffselement eine Kraft (Zugkraft oder Druckkraft) auf das Eingriffselement ausübt, die ein Anpressen des Containerdeckels an die Containerwanne bewirkt. Es ist vorzugsweise so ausgebildet und angeordnet, dass es eine solche Kraft bei in der Schließstellung befindlichem Verschlussmechanismus ausübt. Auf diese Weise kann ein besonders sicherer und dichter Sitz des Deckels auf der Containerwanne erzielt werden, zum Beispiel indem dazwischen eine insbesondere elastische Dichtung angeordnet / ausgebildet ist, die infolge der durch den erfindungsgemäßen Verschlussmechanismus bewirkten Anpresskraft zusätzliche Dichtwirkung zur Verfügung stellt.

Eine weitere Ausführungsform der Erfindung ist dadurch gekennzeichnet, dass das Sperrelement einen Federabschnitt aufweist. Dieser kann im Kraftfluss zwischen dem Containerdeckel und dem Riegelelement angeordnet sein. Er kann dann in vorteilhafter Weise bei in der Schließstellung befindlichem Verschlussmechanismus eine Kraft auf das Riegelelement ausüben, die ein Anpressen des Riegelelements gegen das Eingriffselement bewirkt. Diese Kraft ist dann der durch den Verschlussmechanismus ausgeübten Anpresskraft zwischen Containerdeckel und Containerwanne entgegengerichtet. Ein besonders fester und sicherer Sitz sowohl zwischen Deckel und Container als auch zwischen Riegelelement und Eingriffselement ist ein besonderer Vorteil dieser Ausführungsform. Außerdem kann bei dieser Ausführungsform der Federabschnitt bzw. das Sperrelement eine Art Ausgleichselement zur Verfügung stellen, das bewirkt, dass eine in der Schließstellung zwischen dem Riegelelement und dem Eingriffselement ausgeübte Kraft einen bestimmungsgemäßen Maximalwert nicht übersteigt. Dieser Maximalwert ist durch die Federcharakteristik und/oder Dämpfercharakteristik des Federabschnitts bzw. des Sperrelements bestimmt und kann individuell derart ausgewählt werden / sein, dass eine kritische Belastung von Containerwanne und/oder Containerdeckel und/oder Verschlussmechanismus nicht erreicht wird. Bei dieser Ausführungsform ist es vorteilhaft, wenn das Riegelelement quer zur Deckelebene schwenkbar ist.

Nach einer weiteren Ausführungsform ist das Sperrelement durch eine Blattfeder ausgebildet. Zumindest kann der Federabschnitt des Sperrelements durch eine Blattfeder ausgebildet sein. Bei einem solchen blattfederartigen Sperrelement können die Raststruktur und der Federabschnitt besonders effizient und kompakt in Form eines einteiligen Bauteils, insbesondere Blechformteils aus einem Federmaterial, wie einem Federstahl, ausgebildet sein.

In einer weiteren Ausführungsform kann das Sperrelement ein positionsdefiniert am Containerdeckel angeordneter Kipphebel sein. Dieser kann insbesondere relativ zum Riegelelement zwischen einer dieser in der Schließstellung sperrenden Sperrstellung und einer Freigabestellung schwenkbar sein. Der Kipphebel ist vorzugsweise in die Sperrstellung vorgespannt, zum Beispiel mittels eines gesonderten Vorspannelements in Form einer Feder / Schraubenfeder / Blattfeder. Es ist von besonderem Vorteil, wenn der Kipphebel durch eine vom Containerdeckel und von der Containerwanne fortweisende Betätigung aus der Sperrstellung in seine Freigabestellung überführbar ist, da auf diese Weise ein versehentliches Entsperren des Verschlussmechanismus durch Aufeinanderstapeln von mehreren Sterilcontainern aufeinander in einfacher und sicherer Weise verhindert werden kann.

Zusammenfassend kann man sagen, dass die Erfindung insbesondere die folgenden Vorteile bewirken kann:
- Sie stellt eine Verschlusseinheit zum Verschließen eines Behälteroberteils / Containerdeckel mit einem Behälterunterteil / Containerwanne zur Verfügung, wobei vorzugsweise an einer Stirnseite des Behälterunterteils /Containerdeckels trotz der Verschlusseinheit eine möglichst große Fläche, beispielsweise zur Kennzeichnung des Behälters, frei ist und zur Verfügung steht.
- Durch Greifen/Umgreifen oder Ziehen an der Behälterstirnseite besteht dabei insbesondere nicht unbedingt eine Gefahr, dass sich die Verschlusseinheit versehentlich öffnet oder dass eine versehentliche Betätigung stirnseitig erfolgen kann.
- Durch die Wirkung des Sperrelements / Ausgleichselements / Federabschnitts kann die Verschlusseinheit außerdem in vorteilhafter Weise Toleranzen zwischen dem Behälteroberteil / Containerdeckel und dem Behälterunterteil / Containerwanne ausgleichen. Das Behälteroberteil / Containerdeckel kann dabei insbesondere mit einer definierten Mindestkraft auf das Behälterunterteil / Containerwanne gedrückt werden.
- Im Rahmen einer Ausführungsform der Erfindung kann eine Drehbewegung eines Klappbügels / Betätigungselements der Verschlusseinheit in eine Bahnkurve des Riegelelements / Schiebers transformiert werden, bei der zunehmend die Kraft, welche vom Verschluss auf die Behälterdichtung ausgeübt wird, ansteigen kann.
- Bei Vorliegen eines Überdrucks im Behälterinneren in Relation zum Behälteräußeren (oder anders ausgedrückt bei einem Unterdruck des Behälteräußeren in Relation zum Behälterinneren) kann bei einer Ausführungsform eine Relativbewegung des Behälteroberteils / Containerdeckels in Relation zum Behälterunterteil / Containerwanne stattfinden. Dadurch kann in vorteilhafter Weise ein Bypass zwischen dem Behälteroberteil / Containerdeckel und dem Behälterunterteil / Containerwanne freigegeben werden.
- Ein Betätigungselement / Klappbügel zum Öffnen kann sich insbesondere an der Oberseite des Behälteroberteils / Containerdeckels befinden. Dies führt zu optimierten Platzverhältnissen stirnseitig und entsprechenden Möglichkeiten zur stirnseitigen Kennzeichnung der Behälter / Container.
- Der Abstand vom Klappbügel / Betätigungselement zur Behälteröffnung ist derart bemessen, dass ausreichend Abstand zum Behälterinneren gewährleistet ist, was zum Beispiel für eine sogenannte aseptische Bereitstellung im OP von Wichtigkeit ist. Sie verbessert damit eine aseptische Bereitstellung des Inhalts des Behälters / Containers.
- Bei geschlossenem Verschluss ist dieser in der Endposition / Schließstellung stabilisiert / fixiert / gesichert / gesperrt.
- Ein versehentliches / ungewolltes Öffnen des Verschlusses kann einfach und sicher vermieden / verhindert werden.
- Aus der Sperrstellung ist durch eine Betätigung / Entsperrung des Sperrelements ein besonders einfaches / nutzerfreundliches Öffnen des Verschlusses möglich.

Weitere Merkmale und Vorteile der vorliegenden Erfindung ergeben sich aus der folgenden beispielhaften und nicht beschränkenden Beschreibung der Figuren. Diese sind lediglich schematischer Natur und dienen nur dem Verständnis der Erfindung. Dabei zeigen:
Fig. 1 eine perspektivische Ansicht eines Verschlussmechanismus gemäß einem ersten Ausführungsbeispiel der Erfindung in der Offenstellung, also im vollständig unverschlossenen Zustand,
Fig. 2 eine vergrößerte Detailansicht des Verschlussmechanismus der Figur 1,
Fig. 3 eine perspektivische Ansicht des Verschlussmechanismus der Figuren 1 und 2 in der Schließstellung, also vollständig verschlossenem Zustand,
Fig. 4 eine vergrößerte Detailansicht des Verschlussmechanismus der Figur 3,
Fig. 5 eine perspektivische Ansicht des Verschlussmechanismus der Figuren 1 bis 4 mit einer Abdeckkappe,
Fig. 6 eine perspektivische Ansicht eines zweiten Ausführungsbeispiels in der Offenstellung, also im vollständig unverschlossenen Zustand,
Fig. 7 eine perspektivische Ansicht eines dritten Ausführungsbeispiels in der Offenstellung, also vollständig unverschlossenen Zustand,
Fig. 8 eine perspektivische Ansicht eines vierten Ausführungsbeispiels in der Offenstellung, also im vollständig unverschlossenen Zustand,
Fig. 9 eine perspektivische Ansicht eines fünften Ausführungsbeispiels in der Offenstellung, also im vollständig unverschlossenen Zustand,
Fig. 10 eine perspektivische Ansicht eines sechsten Ausführungsbeispiels, welcher nicht Teil der Offenstellung ist, also im vollständig unverschlossenen Zustand,
Fig. 11 eine Schnittansicht eines Verschlussmechanismus gemäß einem weiteren Ausführungsbeispiel, welcher nicht Teil der Offenstellung ist, und
Fig. 12 eine Schnittansicht des Verschlussmechanismus der Figur 11 in der Schließstellung.

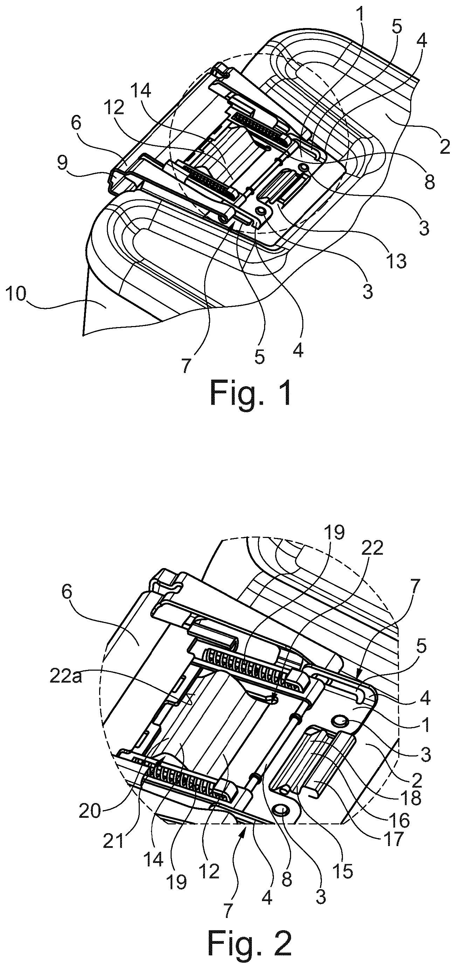

Ein Verschlussmechanismus gemäß Fig. 1 bis 5 besitzt eine Grundplatte 1, die an der Oberseite eines Containerdeckels 2 angeordnet und zum Beispiel mittels Nieten 3 befestigt ist. Die Grundplatte 1 ist ein gestanztes Blechformteil und weist auf beiden Seiten um etwa 90° zur Ebene des Containerdeckels 2 aufgebogene Kragenabschnitte 4 auf. In jeden dieser Kragenabschnitte ist jeweils ein Führungslangloch 5 oder eine Führungsnut 5 eingebracht, dessen / deren Längsrichtung sich im Wesentlichen parallel zur Ebene des Containerdeckels 2 erstreckt.

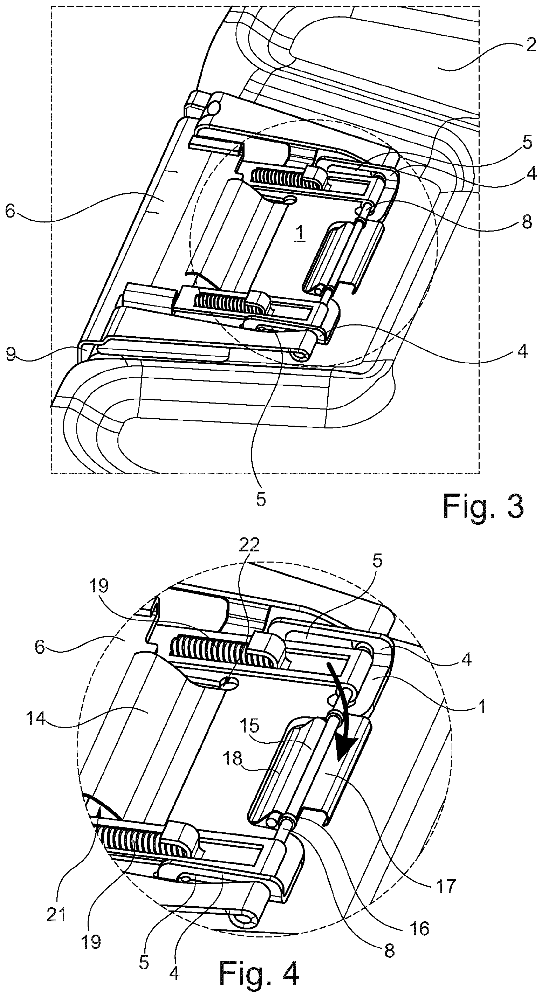

Der Verschlussmechanismus umfasst außerdem ein hakenförmiges Riegelelement 6, zum Beispiel in Form eines Zugbügels / einer Kralle / einer Spannpratze / eines Verschlussschiebers 6. Das Riegelelement 6 ist mittels einer die Führungslanglöcher 5 der Grundplatte 1 durchgreifenden Stange 8 / Achse 8 / Welle 8 an der Oberseite eines Containerdeckels 2 zu dieser parallel verschiebbar gelagert. Es ist außerdem in einem gewissen Bereich um die Längsachse der Stange 8 quer zur Ebene des Containerdeckels 2 schwenkbar. Man kann also sagen, dass die Kragenabschnitte 4 mit den Führungslanglöchern 5 Führungskulissen 7 oder eine Lagerung 7 für das Riegelelement 6 bilden. Durch diese Lagerung 7 kann das Riegelelement 6 zwischen einer Offenstellung, die in den Figuren 1 und 2 gezeigt ist, und einer Schließstellung, die in den Figuren 3, 4 und 5 gezeigt ist, positioniert werden, wobei die Stange 8 in den Führungslanglöchern 7 gleitet.

Das Riegelelement 6 hat / bildet an seinem frei über die Umfangskante des Containerdeckels 2 auskragenden Ende einen Haken- oder Krallenabschnitt 9, der in Richtung Deckelunterseite bzw. einer Containerwanne 10 abgebogen ist. Der Haken- oder Krallenabschnitt 9 ist dafür ausgebildet und vorgesehen, mit einer an der Containerwanne 10 umfangsseitig ausgebildeten Leiste 11 als Eingriffselement 11 in einen diese hinterschneidenden Wirkeingriff zu kommen, wenn sich der Verschlussmechanismus in der Schließstellung befindet. Befindet sich der Verschlussmechanismus in der Freigabestellung oder Offenstellung, stehen der Haken- oder Krallenabschnitt 9 und das Eingriffselement 11 nicht miteinander in Eingriff und der Containerdeckel 2 kann von der Containerwanne 10 abgenommen werden. In der Schließstellung ist der Containerdeckel 2 an der Containerwanne 10 gesichert / befestigt.

Der Verschlussmechanismus der ersten Ausführungsform der Figuren 1 bis 5 besitzt des Weiteren ein Blechformteil 12. Dieses beinhaltet mehrere Funktionen. Zum einen bildet es ein Sperrelement/Sperrabschnitt 13 im Sinne der Erfindung aus. Zum anderen bildet es ein Blattfederelement/Blattfederabschnitt 14 zum Vorspannen / Verspannen des Riegelelements 6 gegenüber dem Eingriffselement 11 / der Leiste 11 der Containerwanne 10. Während das Sperrelement 13 und das Blattfederelement 14 bei dem vorliegenden Ausführungsbeispiel einstückig durch das Blechformteil gebildet sind, können sie im Rahmen der Erfindung auch mehrteilig, insbesondere zweiteilig durch eine Mehrzahl an Blechformteilen ausgebildet sein.

Das Sperrelement 13 ist bei der Ausführungsform der Figuren 1 bis 5 durch einen frei auskragenden Arm des Blechformteils 12 gebildet, der Federeigenschaften aufweist. Es besitzt eine Raststruktur 15, hier in Form einer Vertiefung 15, die eine Anlageschulter 16 ausbildet. Die Breite der Vertiefung 15 ist etwas größer als die Dicke der Stange 8. Das distale freie Ende des Sperrelements 13 ist als Betätigungselement 17 ausgebildet. Auf der dem distalen freien Ende gegenüberliegenden Seite der Vertiefung 15 ist das Sperrelement 13 mit einer Auflaufschräge 18 für die Stange 8 versehen. Infolge der Federeigenschaften des Sperrelements 13 ist dieses in eine von der Grundplatte 1 abstehende Ruheposition vorgespannt. Aus dieser kann es gegen seine Federvorspannung in Richtung hin zu der Grundplatte 1 federnd verlagert werden.

Wenn sich der Verschlussmechanismus und damit das Riegelelement 6 in der Freigabestellung / Offenstellung befinden, sind die Stange 8 und das Sperrelement 13 außer Eingriff. Diese Situation ist in den Figuren 1 und 2 gezeigt. Die Stange 8 ist auf der dem Betätigungselement 17 gegenüberliegenden Seite der Vertiefung 15 und der Auflaufschräge 18 positioniert. Wie insbesondere in den Figuren 3 und 4 dargestellt ist, kommt die Stange 8 bei in der Schließstellung befindlichem Riegelelement 6 in der Vertiefung 15 zu liegen. Bei einer Verbringung des Riegelelements 6 aus der Freigabestellung in die Schließstellung wird die Stange 8 zusammen mit dem Riegelelement 6 verlagert, und zwar in Richtung hin zum Sperrelement 13. Die Stange 8 läuft dabei auf die Auflaufschräge 18 des Sperrelements 13 auf. Da das Riegelelement 6 und die Stange 8 in der Führungskulisse 7 Vorzugsweise parallel zur Ebene des Containerdeckels 2 geführt sind, wird das Sperrelement 13 durch die Stange 8 bei fortgeführter Bewegung in die Schließstellung entgegen seiner Vorspannung in Richtung hin zum Containerdeckel 2 und der Grundplatte 1 verlagert. Man kann auch sagen, dass das Sperrelement 13 in Richtung zur Grundplatte 1 bzw. zum Containerdeckel 2 einfedert. Sobald die Stange 8 in einer Überdeckung mit der Vertiefung 15 gelangt, federt das Sperrelement 13 infolge seiner Federeigenschaften zurück in seine von der Grundplatte beabstandete Ruheposition. Die Stange 8 kommt dabei in der Vertiefung 15 zum Liegen und in Anlage mit der Schulter 16. Das Riegelelement 6 ist somit durch das Sperrelement 13 in der Schließstellung gesichert. Auf diese Weise bilden die Vertiefung 15 mit der Schulter 16 eine Raststruktur und die Stange 8 eine damit zusammenwirkende Gegenraststruktur im Sinne der Erfindung aus.

Der Verschlussmechanismus weist außerdem zwei Schraubenfedern 19 als Federelemente 19 auf, die in die Freigabestellung / Offenstellung vorspannen. Die Schraubenfedern 19 sind jeweils einerseits an dem Riegelelement 6 und andererseits an der Grundplatte 1 abgestützt. Bei einem Verbringen des Riegelelements 6 in die Schließstellung werden die Schraubenfedern 19 gegen ihre Federspannung zusammengepresst. Zum Verbringen des Verschlussmechanismus und des Riegelelements 6 aus der Schließstellung in die Freigabestellung ist eine nutzerseitige Betätigung des Betätigungselements 17 erforderlich, bei der dieses entgegen der Federspannung des Sperrelements 13 in Richtung zur Grundplatte 1 bzw. zum Containerdeckel 2 gedrückt und verlagert wird. Infolge dieser Verlagerung kommen die Schulter 16 und die Stange 8 außer Eingriff und infolge der Spannung der Schraubenfedern 19 wird das Riegelelement 6 aus der Schließstellung zurück in die Freigabestellung verlagert.

Das durch das Blechformteil 12 gebildete Blattfederelement 14 besitzt eine von der Ebene des Containerdeckels 2 fort ragende Auflauffläche 20, die durch einen wellenförmig oder dachförmig verformten Bereich 21 gebildet ist. Dieser dachförmige Bereich 21 ragt ebenfalls von der Ebene des Containerdeckels 2 bzw. der Grundplatte 1 ab und in eine in dem Riegelelement 6 ausgebildete Ausnehmung 22 / ein Fenster 22 hinein. Eine die Ausnehmung 22 begrenzende Kante 22a gelangt bei einem Verbringen des Riegelelements 6 in die Schließstellung mit der Auflauffläche 20 des Bereichs 21 in Wechselwirkung und läuft auf diese auf. Dadurch kommt es zum einen ggf. zu einer elastischen Verformung des dachförmigen Bereichs 21 bzw. der Auflauffläche 20 in Richtung zur Grundplatte und zum andern zu einer Verlagerung des Haken- oder Krallenabschnitt 9, da das Riegelelement 6 nur an seiner distalen freien Seite mittels der Stange 8 und der Führung 7 relativ zur Grundplatte 1 und dem Containerdeckel 2 geführt ist, im Übrigen jedoch um die Stange 8 drehbar ist. Die Kinematik ist dabei derart ausgebildet und abgestimmt, dass ein Verschwenken des Haken- oder Krallenabschnitts 9 fort von der Grundplatte 1 infolge der Wechselwirkung mit der Auflauffläche 20 erst stattfindet oder beginnt, wenn der Haken- oder Krallenabschnitt 9 die Leiste 11 der Containerwanne 10 hintergreift. Auf diese Weise bewirkt der Verschlussmechanismus zum einen eine Verspannung des Haken- oder Krallenabschnitt 9 mit der Leiste 11 (und so einen festen Sitz) und besitzt durch die Elastizität des dachförmigen Bereichs 21 zum anderen eine Art Überlastsicherung, die ein Anpressen des Containerdeckels 2 an die Containerwanne 10 durch die dabei wirkende Federkraft definiert.

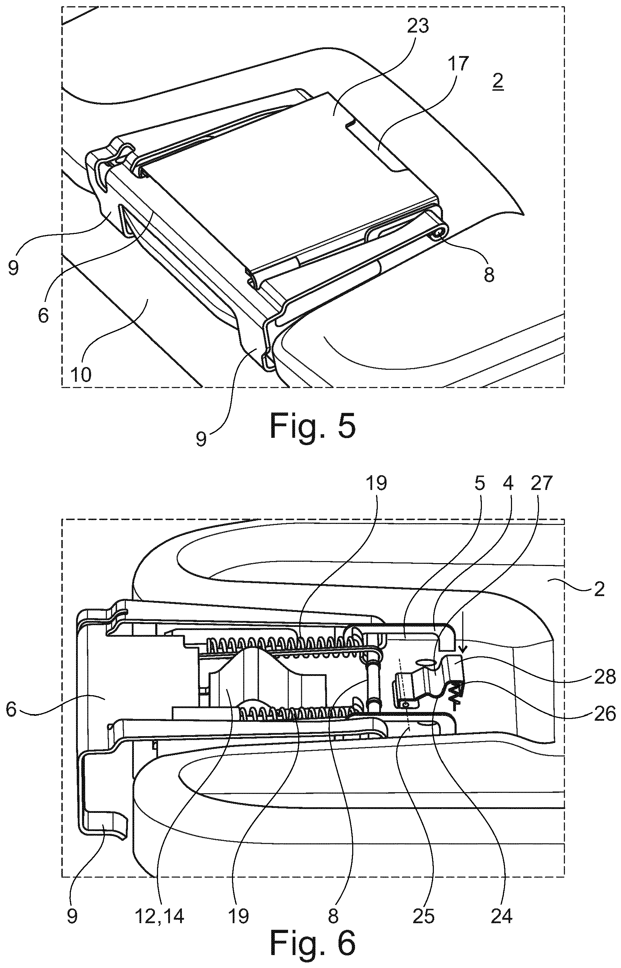

In Figur 5 ist dargestellt, dass der in den Figuren 1 bis 4 gezeigte Verschlussmechanismus größtenteils mittels einer Abdeckung 23 abgedeckt ist, die lediglich das Betätigungselement 17 frei lässt.

Figur 6 zeigt in einer perspektivischen Ansicht ein weiteres Ausführungsbeispiel des Verschlussmechanismus in der Offenstellung, der sich von der Ausführungsform der Figuren 1 bis 5 lediglich durch die Ausbildung des Sperrelements unterscheidet. Bei dieser Ausführungsform bildet ein Blechformteil 12 nur das Blattfederelement 14 aus. Ein Kipphebel 24 ist separat von dem Blechformteil 12 ausgebildet und stellt als Sperrelement im Sinne der Erfindung dar. Der Kipphebel 24 ist positionsfest um eine Schwenkachse 25 schwenkbar an der Grundplatte 1 (oder am Containerdeckel 2) angeordnet. Er ist zwischen einer Ruheposition / Sperrposition, in die er mittels einer Druckfeder 26 vorgespannt ist, und einer Freigabeposition, in die er durch eine nutzerseitige Betätigung gegen die Kraft der Druckfeder 26 gebracht werden kann, positionierbar. Der Kipphebel 24 weist eine Vertiefung 27 oder einen Haken 27 auf, die / der in die von der Grundplatte abgewandten Richtung offen und zur Aufnahme der / Wechselwirkung mit der Stange 8 ausgebildet und bestimmt ist, wenn sich das Riegelelement 6 in der Schließstellung befindet. Das von der Schwenkachse 25 entfernte Ende des Schwenkhebels 24 ist als Betätigungselement 28 zur Druckbetätigung durch einen Nutzer ausgebildet. Der Kipphebel 24 kann demnach das Riegelelement 6 in der Sperrposition sperren und gibt es in der Freigabeposition zur Bewegung frei.

Bei Verbringen des Riegelelements 6 in die Schließstellung läuft die Stange 8 auf den Kipphebel 24 auf. Dieser verschwenkt infolgedessen gegen die Kraft der Druckfeder 26 in Richtung des Containerdeckels 2 / der Grundplatte1, bis die Stange 8 mit der Vertiefung 27 in Überdeckung steht und der Kipphebel 24 durch die Wirkung der Druckfeder 26 zurück in seine Ruheposition schwenkt. In dieser liegt die Stange 8 in der Vertiefung 27, so dass das Riegelelement 6 in der Schließstellung gesperrt ist. Durch eine Druckbetätigung des Betätigungselements 28 in Richtung der Grundplatte 1 gibt die Vertiefung 27 die Stange 8 frei und das Riegelelement 6 gelangt aufgrund der Vorspannung durch die Schraubenfedern 19 aus der Schließstellung automatisch zurück in die Freigabestellung.

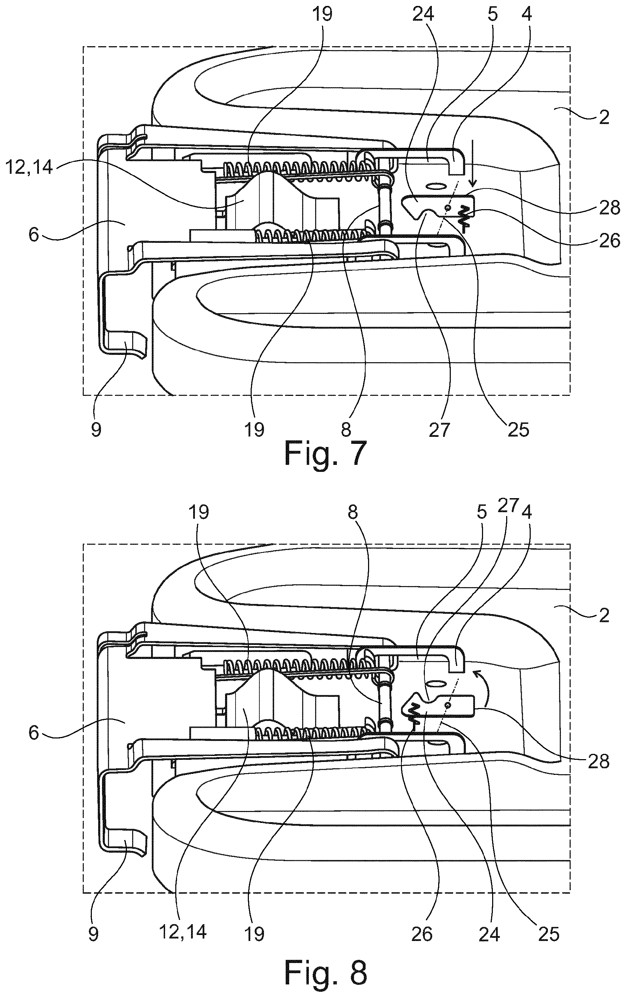

Figur 7 zeigt eine perspektivische Ansicht des Verschlussmechanismus gemäß einem weiteren Ausführungsbeispiel in der Offenstellung. Dieses gleicht im Wesentlichen dem der Figur 6, unterscheidet sich aber in der Anordnung der Schwenkachse 25 und der Ausrichtung der in Richtung der Grundplatte offenen Vertiefung 27 des Kipphebels 24.

Figur 8 zeigt ein weiteres Ausführungsbeispiel, bei der die Vertiefung 27 des Kipphebels 24 noch oben offen ist (also auf der von der Grundplatte 1 abgewandten Seite offen ist), und die Druckfeder 26 und die Vertiefung 27 auf der einen Seite der Schwenkachse 25 und das Betätigungselement 28 auf Seite der Schwenkachse 25 angeordnet sind. Es ist ein besonderer Vorteil dieser Ausführungsform, dass das Betätigungselement 28 nicht in Richtung der Grundplatte 1 und des Containerdeckels 2 druckbetätigbar ist, sondern durch eine Zugbetätigung in einer Richtung fort von der Grundplatte 1 und dem Containerdeckel 2 zu betätigen ist. Auf diese Weise wird vermieden, dass es bei einem Aufeinanderstapeln mehrerer Sterilcontainer 10 aufeinander zu einer versehentlichen Betätigung des Betätigungselements 28 und einer Entsperrung des Riegelelements 6 und des Verschlussmechanismus kommen kann.

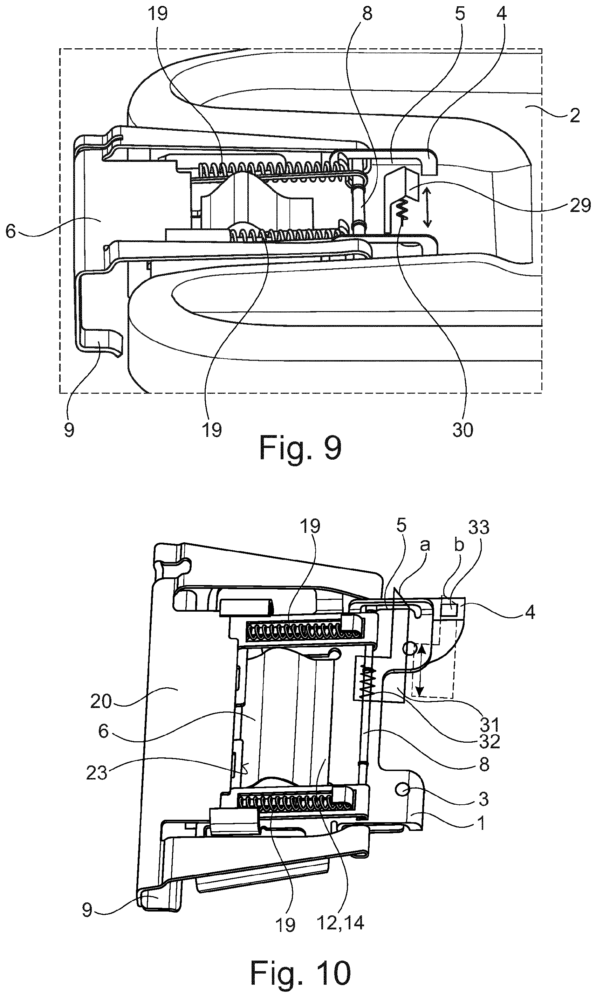

Figur 9 zeigt eine perspektivische Ansicht eines weiteren Ausführungsbeispiels der Erfindung in der Offenstellung. Bei dieser ist anstelle eines Kipphebels 24 ein Druckknopf 29 als Sperrelement vorgesehen, der für ein Zusammenwirken mit der Stange in einer bereits mit Bezug zu den übrigen Ausführungsbeispielen beschriebenen Weise ausgebildet und bestimmt ist und mit einer Druckfeder 30 in eine die Stange 8 sperrende Sperrstellung vorgespannt ist.

Figur 10 zeigt ein Ausführungsbeispiel, welcher nicht Teil der Erfindung ist, bei der das Sperrelement in Form eines quer zur Ebene des Containerdeckels 2 und der Grundplatte 1 positionierbaren Sperrelements 31 gebildet ist. Das Sperrelement 31 ist auf der Stange 8 in deren Längsrichtung relativpositionierbar zwischen einer Sperrstellung und einer Freigabestellung gelagert. Es ist außerdem mittels es Federelements 32 in die Sperrstellung vorgespannt. Im Vergleich zu den übrigen Ausführungsbeispielen ist der Kragenabschnitt 4 der Grundplatte 1 verlängert ausgebildet und weist eine Sperrausnehmung 33 auf, in die das Sperrelement 31 bei in der Schließstellung befindlichem Verschlussmechanismus / Riegelelement 6 aufgrund der Vorspannung durch das Federelement 32 automatisch eingreift / eingreifen kann. Zum Verbringen des Verschlussmechanismus aus der Schließstellung in die Freigabestellung wird das Sperrelement 31 nutzerzeitig gegen die Federvorspannung betätigt und außer Eingriff der Sperrausnehmung 33 gebracht, so dass das Riegelelement 6 durch die Spannung der Schraubenfedern 19 eigenständig in die Freigabestellung wechselt. Während in Figur 10 nur ein Sperrelement 31 auf einer Seite des Verschlussmechanismus dargestellt ist, kann eine Variante dieses Ausführungsbeispiels zwei derartige Sperrelemente 31 aufweisen, die beiderseits des Verschlussmechanismus angeordnet sind.

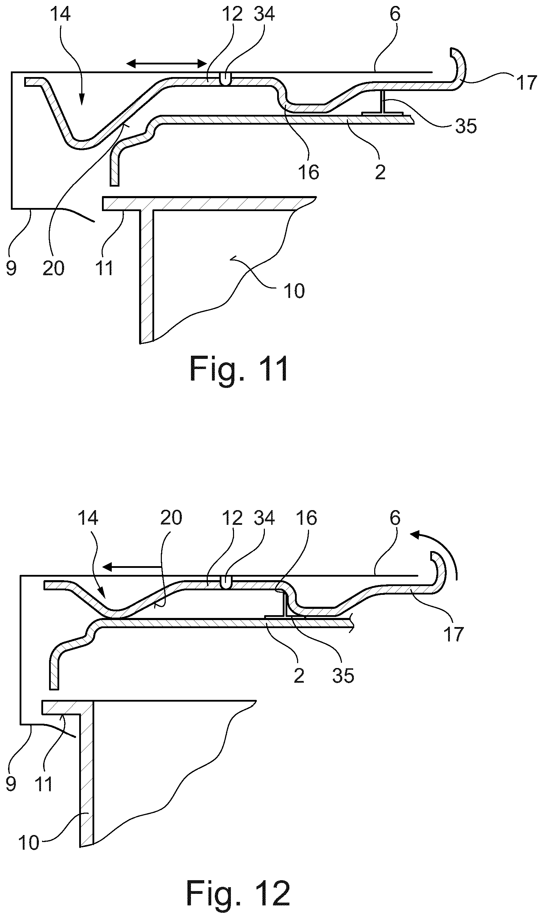

Figur 11 zeigt eine Schnittansicht eines weiteren Ausführungsbeispiels, welcher nicht Teil der Offenstellung ist, das sich von dem Ausführungsbeispiel der Figuren 1 bis 5 dadurch unterscheidet, dass das Blechformteil 12 nicht ortsfest am Containerdeckel 2, sondern ortsfest am Riegelelement 6 angeordnet ist. Figur 12 zeigt eine Schnittansicht des Verschlussmechanismus der Figur 11 in der Schließstellung. Das Blechformteil 12 kann zum Beispiel über eine Niet- oder Schraubverbindung 34 oder eine stoffschlüssige Verbindung 34 (Punktschweißung, etc.) mit dem Riegelelement 6 verbunden sein. Am Containerdeckel 2 oder an der Grundplatte 1 kann ein Rastelement 35 angeordnet sein, dass für ein Zusammenwirken mit dem Sperrelement 13 des Blechformteils 12 ausgebildet ist. Im Übrigen entspricht / gleicht das Ausführungsbeispiel der Figuren 11 und 12 dem der Figuren 1 bis 5, so dass auf die dortige Beschreibung weiterer Einzelheiten Bezug genommen wird. Ein besonderer Vorteil dieses Ausführungsbeispiels ist, dass das Blechformteil 12 unabhängig von der Funktionsstellung des Riegelelements 6 von diesem nahezu komplett abgedeckt und geschützt ist und dass ein Entsperren des Sperrelements 13 durch eine Zugbetätigung des Betätigungselements 17 erfolgt (anders als bei dem Ausführungsbeispiel der Figuren 1 bis 5, bei dem eine Druckbetätigung in Richtung des Containerdeckels erfolgt), so dass ein unbeabsichtigtes Entsperren bei einem Aufeinanderstapeln mehrerer Sterilcontainer sicher vermieden werden kann.

### Bezugszeichenliste

- 1: Grundplatte, Grundblech
- 2: Containerdeckel
- 3: Niet
- 4: Kragenabschnitte
- 5: Führungslangloch, Führungsnut
- 6: Riegelelement
- 7: Führungskulisse, Lagerung
- 8: Stange, Achse, Welle
- 9: Haken- oder Krallenabschnitt
- 10: Containerwanne
- 11: Eingriffselement, Leiste
- 12: Blechformteil
- 13: Sperrelement
- 14: Federelement, Blattfederelement
- 15: Vertiefung
- 16: Schulter
- 17: Betätigungselement
- 18: Auflaufschräge
- 19: Federelement, Schraubenfeder
- 20: Auflauffläche
- 21: dachförmiger Bereich
- 22: Ausnehmung, Fenster
- 22a: Kante
- 23: Abdeckung
- 24: Kipphebel
- 25: Schwenkachse
- 26: Druckfeder
- 27: Vertiefung, Haken
- 28: Betätigungselement
- 29: Druckknopf
- 30: Druckfeder
- 31: Sperrelement
- 32: Federelement
- 33: Sperrausnehmung
- 34: Verbindung, Niet- oder Schraubverbindung
- 35: Rastelement

## Patentansprüche

1. Verschlussmechanismus für einen Sterilcontainer mit einer Containerwanne (10) und einem mittels des Verschlussmechanismus mit der Containerwanne (10) verriegelbaren Containerdeckel (2),
wobei der Verschlussmechanismus ein zur verschiebbaren Lagerung am Containerdeckel (2) oder an der Containerwanne (10) vorgesehenes und ausgebildetes Riegelelement (6) zum hinterschneidenden Eingriff mit einem eine Hinterschneidung ausbildenden Eingriffselement (11) an der Containerwanne (10) bzw. am Containerdeckel (2) aufweist,
wobei das Riegelelement (6) in eine Schließstellung, in der es mit dem Eingriffselement (11) in hinterschneidendem Wirkeingriff steht, und in eine Freigabestellung positionierbar bzw. verschiebbar ist, in der das Riegelelement (6) und das Eingriffselement (11) außer Eingriff sind,
wobei der Verschlussmechanismus ein mit dem Riegelelement (6) wirkverbundenes oder wirkverbindbares Sperrelement (13, 24, 29) aufweist, das derart in den Verschlussmechanismus integriert ist oder mit diesem zusammenwirkt, dass es den Verschlussmechanismus bei Erreichen der Schließstellung in dieser Schließstellung sperrt, wobei das Sperrelement (13, 24, 29) eine Raststruktur (15, 16), insbesondere in Form einer Schulter oder eines Hakens, aufweist, die bei in der Schließstellung befindlichem Verschlussmechanismus mit einer an dem Riegelelement (6) als eine Stange angeordnete oder ausgebildete Gegenraststruktur (8) in Eingriff gelangt.

2. Verschlussmechanismus nach Anspruch 1, **gekennzeichnet durch** ein Federelement bzw. Rückstellfeder (19), das den Verschlussmechanismus, insbesondere das Riegelelement (6), in die Freigabestellung vorspannt.

3. Verschlussmechanismus nach Anspruch 1 oder 2, **dadurch gekennzeichnet, dass** das Sperrelement (13, 24, 29) elastisch ausgebildet und/oder angeordnet ist, insbesondere ein Blattfederelement (12) ist oder durch ein solches gebildet ist, oder das Sperrelement (13, 24, 29) elastisch vorgespannt ausgebildet ist, insbesondere ein elastisch vorgespanntes Hebelelement oder Kipphebel (24) oder ein elastisch vorgespannter Druckknopf (29) ist, wobei das Sperrelement (13, 24, 29) insbesondere derart ausgebildet und angeordnet ist, dass es bei Erreichen der Schließstellung des Verschlussmechanismus aufgrund seiner Vorspannung eine den Verschlussmechanismus in der Schließstellung sperrende Sperrstellung einnimmt und durch eine nutzerseitige Betätigung entgegen der Vorspannung aus der Sperrstellung in eine den Verschlussmechanismus freigebende Freigabestellung überführbar ist.

4. Verschlussmechanismus nach einem der vorstehenden Ansprüche, **dadurch gekennzeichnet, dass** das Riegelelement (6) derart ausgebildet und angeordnet ist, dass es im Wirkeingriff mit dem Eingriffselement (11) und insbesondere bei in der Schließstellung befindlichem Verschlussmechanismus eine Zugkraft oder eine Druckkraft auf das Eingriffselement (11) ausübt, die ein Anpressen des Containerdeckels (2) an die Containerwanne (10) bewirkt.

5. Verschlussmechanismus nach einem der vorstehenden Ansprüche, **dadurch gekennzeichnet, dass** dieser einen schwenkbar am Containerdeckel (2) bzw. an der Containerwanne (10) lagerbaren Betätigungshebel aufweist, der derart mit dem Riegelelement (6) direkt oder indirekt gekoppelt ist, dass das Riegelelement (6) mittels des Betätigungshebels in die Schließstellung positionierbar ist.

6. Verschlussmechanismus nach einem der vorstehenden Ansprüche, **dadurch gekennzeichnet, dass** der Verschlussmechanismus ein Federelement (14), insbesondere das Sperrelement (13, 24, 29) einen Federabschnitt (14), aufweist, der im Kraftfluss zwischen dem Containerdeckel (2) und dem Riegelelement (6) angeordnet ist und bei in der Schließstellung befindlichem Verschlussmechanismus auf das Riegelelement (6) eine Kraft ausübt, die ein Anpressen des Riegelelements (6) gegen das Eingriffselement (11) bewirkt.

7. Verschlussmechanismus nach einem der vorstehenden Ansprüche, **dadurch gekennzeichnet, dass** das Sperrelement (13), zumindest der Federabschnitt (14) des Sperrelements (13), eine Blattfeder (12) ist oder durch eine Blattfeder (12) ausgebildet ist.

8. Verschlussmechanismus nach Anspruch 7, **dadurch gekennzeichnet, dass** das Sperrelement (24) ein positionsdefiniert am Containerdeckel (2) angeordneter Kipphebel (24) ist, der relativ zum Riegelelement (6) zwischen einer dieses in der Schließstellung sperrenden Sperrstellung und einer Freigabestellung schwenkbar ist, wobei der Kipphebel (24) vorzugsweise in die Sperrstellung vorgespannt ist.

9. Verschlussmechanismus nach einem der vorstehenden Ansprüche, **dadurch gekennzeichnet, dass** das Sperrelement (13, 24, 29) durch eine vom Containerdeckel fortweisende Betätigung aus der Sperrstellung in seine Freigabestellung überführbar ist.

10. Sterilcontainer mit einem Containerdeckel (2) und einer Containerwanne (10) und einem Verschlussmechanismus nach einem der vorstehenden Ansprüche wobei der Verschlussmechanismus die folgenden Teile hat:
- einen spannpratzen- oder krallenartigen Schieber/Riegelelement (6), der auf dem Containerdeckel (2) verschiebbar gelagert ist, wobei dessen Krallenabschnitt (9) seitlich über den Containerdeckelrand ragt,
- mindestens ein Druckfederelement (14), welches zwischen dem Schieber/Riegelelement (6) und dem Containerdeckel (2) angeordnet ist und welches den Schieber/Riegelelement (6) mit zunehmender Verschiebebewegung aus seiner Freigabeposition in seine Schließposition immer stärker vom Containerdeckel (2) wegdrückt und
- mindestens eine Rückstellfeder (19), welche den Schieber/Riegelelement (6) in Richtung hin zu seiner Freigabeposition vorspannt, wobei
- der Schieber/Riegelelement (6) oder das Druckfederelement (14) mit einem vorzugsweise manuell betätigbaren Halteelement/Halteabschnitt versehen oder ausgebildet ist, das mit einem vorzugsweise manuell betätigbaren Sperr-/Eingriffselement am bzw. auf Seiten des Containerdeckels (2) bei Erreichen der Schließposition in Halteingriff bringbar ist, um den Schieber/Riegelelement (6) entgegen der Vorspannkraft der Rückstellfeder (19) in Schließposition zu halten, in welcher der Krallenabschnitt (9) einen Containerwannenrand untergreift und den Containerdeckel (2) infolge der Federkraft des mindestens einen Druckfederelements (14) gegen den Containerwannenrand anzieht, so lange, bis das Sperr-/Eingriffselement auf Seiten des Containerdeckels (2) oder das Halteelement/Halteabschnitt auf Seiten des Druckfederelements (14) manuell für ein Freigeben des Schiebers/Riegelelements (6) betätigt wird und der Schieber/Riegelelement (6) aufgrund der Rückstellfeder (19) in seine Freigabeposition zurückschnellt.

## Claims

1. A closing mechanism for a sterile container having a container pan (10) and a container lid (2) lockable to the container pan (10) via the closing mechanism,
wherein the closing mechanism comprises a bar element (6) provided and configured for displaceable support on the container lid (2) or on the container pan (10) for undercutting engagement with an engagement element (11) forming an undercut on the container pan (10) or on the container lid (2), respectively,
wherein the bar element (6) is positionable or displaceable into a closed position, in which it is in undercutting operative connection with the engagement element (11), and into a release position, in which the bar element (6) and the engagement element (11) are disengaged,
wherein
the closing mechanism has a locking element (13, 24, 29) which is operatively connected or operatively connectable to the bar element (6) and which is integrated into the closing mechanism or interacts therewith in such a way that it locks the closing mechanism in this closed position when the closed position is reached, wherein the locking element (13, 24, 29) has a latching structure (15, 16), in particular in the form of a shoulder or a hook, which, when the closing mechanism is in the closed position, engages with a counter latching structure (8) arranged or formed on the bar element (6) as a rod and thus locks the closing mechanism in the closed position.

2. The closing mechanism according to claim 1, **characterized by** a spring element or return spring (19) that pre-tensions the closing mechanism, in particular the bar element (6), into the release position.

3. The closing mechanism according to claim 1 or 2, **characterized in that** the locking element (13, 24, 29,) is configured and/or arranged to be elastic, is in particular a leaf spring element (12) or is formed by such an element, or the locking element (13, 24, 29) is configured to be elastically pre-tensioned, is in particular an elastically pre-tensioned lever element or rocker lever (24) or an elastically pre-tensioned press button (29), wherein the locking element (13, 24, 29) is configured and arranged in particular in such a way that, when the closing mechanism reaches the closed position, it assumes a locking state locking the closing mechanism in the closed position due to its pre-tension and can be transferred from the locking state to a release position releasing the closing mechanism by actuation by the user against the pre-tension.

4. The closing mechanism according to one of the preceding claims, **characterized in that** the bar element (6) is configured and arranged in such a way that, in operative connection with the engagement element (11) and in particular when the closing mechanism is in the closed position, it exerts a tensile force or a compressive force on the engagement element (11) that causes the container lid (2) to be pressed against the container pan (10).

5. The closing mechanism according to one of the preceding claims, **characterized in that** it has an actuating lever which can be pivotably mounted on the container lid (2) or on the container pan (10), respectively, and which is coupled directly or indirectly to the bar element (6) in such a way that the bar element (6) can be positioned into the closed position via the actuating lever.

6. The closing mechanism according to one of the preceding claims, **characterized in that** the closing mechanism has a spring element (14), in particular the locking element (13, 24, 29) has a spring portion (14), which is arranged in the force flow between the container lid (2) and the bar element (6) and exerts a force on the bar element (6) when the closing mechanism is in the closed position, which causes the bar element (6) to be pressed against the engagement element (11).

7. The closing mechanism according to one of the preceding claims, **characterized in that** the locking element (13), at least the spring portion (14) of the locking element (13), is a leaf spring (12) or is configured by a leaf spring (12).

8. The closing mechanism according to claim 7, **characterized in that** the locking element (24) is a rocker lever (24) arranged in a defined position on the container lid (2), which is pivotable relative to the bar element (6) between a locking state that locks it in the closed position and a release position, wherein the rocker lever (24) is preferably pre-tensioned in the locking state.

9. The closing mechanism according to one of the preceding claims, **characterized in that** the locking element (13, 24, 29) can be transferred from the locking state to its release position by an actuation pointing away from the container lid.

10. A sterile container comprising a container lid (2) and a container pan (10) and a closing mechanism according to one of the preceding claims wherein the closing mechanism has the following parts:
- a clamping jaw-like or claw fastener-like slider/bar element (6) which is displaceably mounted on the container lid (2), wherein its claw fastener portion (9) projects laterally over the container lid edge,
- at least one compression spring element (14), which is arranged between the slider/bar element (6) and the container lid (2) and which presses the slider/bar element (6) increasingly away from the container lid (2) with increasing translational movement from its release position into its closed position, and
- at least one return spring (19) which pre-tensions the slider/bar element (6) in the direction of its release position, wherein
- the slider/bar element (6) or the compression spring element (14) is provided with or configured with a preferably manually operable retaining element/retaining portion which can be brought into retaining engagement with a preferably manually operable locking/engagement element at or respectively on the side of the container lid (2) upon reaching the closed position, in order to hold the slider/bar element (6) in the closed position against the pre-tensioning force of the return spring (19), in which the claw fastener portion (9) engages under a container pan edge and pulls the container lid (2) against the container pan edge as a result of the spring force of the at least one compression spring element (14), until the locking/engagement element on the side of the container lid (2) or the retaining element/retaining portion on the side of the compression spring element (14) is manually actuated for releasing the slider/bar element (6), and the slider/bar element (6) springs back into its release position due to the return spring (19).

## Revendications

1. Mécanisme de fermeture pour un récipient stérile avec un bac de récipient (10) et un couvercle de récipient (2) verrouillable au moyen du mécanisme de fermeture avec le bac de récipient (10),
dans lequel le mécanisme de fermeture présente un élément de verrou (6) prévu et formé pour le logement mobile au niveau du couvercle de récipient (2) ou au niveau du bac de récipient (10) pour la prise en contre-dépouille avec un élément de prise (11) formant une contre-dépouille au niveau du bac de récipient (10) ou au niveau du couvercle de récipient (2),
dans lequel l'élément de verrou (6) peut être positionné ou déplacé dans une position de fermeture dans laquelle il est en prise active en contre-dépouille avec l'élément de prise (11) et dans une position de libération dans laquelle l'élément de verrou (6) et l'élément de prise (11) sont désengagés,
dans lequel le mécanisme de fermeture présente un élément de blocage (13, 24, 29) relié ou pouvant être relié activement à l'élément de verrou (6), élément de blocage qui est intégré dans le mécanisme de fermeture ou coagit avec celui-ci de telle manière qu'il bloque le mécanisme de fermeture lorsque la position de fermeture est atteinte dans cette position de fermeture, dans lequel l'élément de blocage (13, 24, 29) présente une structure d'encliquetage (15, 16), en particulier sous la forme d'un épaulement ou d'un crochet qui parvient en prise lorsque le mécanisme de fermeture se trouve dans la position de fermeture avec une structure d'encliquetage antagoniste (8) formée ou disposée comme une tige au niveau de l'élément de verrou (6).

2. Mécanisme de fermeture selon la revendication 1, **caractérisé par** un élément de ressort ou ressort de rappel (19) qui précontraint le mécanisme de fermeture, en particulier l'élément de verrou (6) dans la position de libération.

3. Mécanisme de fermeture selon la revendication 1 ou 2, **caractérisé en ce que** l'élément de blocage (13, 24, 29) est formé et/ou disposé de manière élastique, en particulier est un élément de ressort à lames (12) ou est formé par un tel élément, ou l'élément de blocage (13, 24, 29) est formé précontraint élastiquement, en particulier est un élément de levier ou levier de basculement (24) précontraint élastiquement ou un bouton pression (29) précontraint élastiquement, dans lequel l'élément de blocage (13, 24, 29) est formé et disposé en particulier de telle manière qu'il occupe une position de blocage bloquant le mécanisme de fermeture dans la position de fermeture lorsque la position de fermeture du mécanisme de fermeture est atteinte en raison de sa précontrainte et puisse être transféré par un actionnement côté utilisateur à l'encontre de la précontrainte depuis la position de verrouillage dans une position de libération libérant le mécanisme de fermeture.

4. Mécanisme de fermeture selon l'une quelconque des revendications précédentes, **caractérisé en ce que** l'élément de verrou (6) est formé et disposé de telle manière qu'il exerce en prise active avec l'élément de prise, (11) et en particulier lorsque le mécanisme de fermeture se trouve dans la position de fermeture, une force de traction ou une force de compression sur l'élément de prise (11) qui provoque un pressage du couvercle de récipient (2) contre le bac de récipient (10).

5. Mécanisme de fermeture selon l'une quelconque des revendications précédentes, **caractérisé en ce que** celui-ci présente un levier d'actionnement pouvant être logé de manière pivotante au niveau du couvercle de récipient (2) ou au niveau du bac de récipient (10), levier qui est couplé directement ou indirectement à l'élément de verrou (6) de telle manière que l'élément de verrou (6) puisse être positionné dans la position de fermeture au moyen du levier d'actionnement.

6. Mécanisme de fermeture selon l'une quelconque des revendications précédentes, **caractérisé en ce que** le mécanisme de fermeture présente un élément de ressort (14), en particulier l'élément de blocage (13, 24, 29) présente une section de ressort (14) qui est disposée en flux de force entre le couvercle de récipient (2) et l'élément de verrou (6) et exerce une force sur l'élément de verrou (6) lorsque le mécanisme de fermeture se trouve dans la position de fermeture qui provoque un pressage de l'élément de verrou (6) contre l'élément de prise (11).

7. Mécanisme de fermeture selon l'une quelconque des revendications précédentes, **caractérisé en ce que** l'élément de blocage (13), au moins la section de ressort (14) de l'élément de blocage (13), est un ressort à lames (12) ou est formé par un ressort à lames (12).

8. Mécanisme de fermeture selon la revendication 7, **caractérisé en ce que** l'élément de blocage (24) est un levier de basculement (24) disposé en position définie au niveau du couvercle de récipient (2), levier qui peut être pivoté par rapport à l'élément de verrou (6) entre une position de blocage le verrouillant dans la position de fermeture et une position de libération, dans lequel le levier de basculement (24) est de préférence précontraint dans la position de blocage.

9. Mécanisme de fermeture selon l'une quelconque des revendications précédentes, **caractérisé en ce que** l'élément de blocage (13, 24, 29) peut être transféré par un actionnement s'éloignant du couvercle de récipient depuis la position de blocage dans sa position de libération.

10. Récipient stérile avec un couvercle de récipient (2) et un bac de récipient (10) et un mécanisme de fermeture selon l'une quelconque des revendications précédentes, dans lequel le mécanisme de fermeture présente les pièces suivantes :
- un coulisseau/élément de verrou (6) de type griffe de serrage ou griffe qui est logé de manière mobile sur le couvercle de récipient (2), dans lequel sa section de griffe (9) dépasse latéralement du bord de couvercle de récipient,
- au moins un élément de ressort de pression (14) qui est disposé entre le coulisseau/l'élément de verrou (6) et le couvercle de récipient (2) et qui repousse le coulisseau/l'élément de verrou (6) toujours plus fortement du couvercle de récipient (2) avec un mouvement de coulissement croissant de sa position de libération dans sa position de fermeture et
- au moins un ressort de rappel (19) qui précontraint le coulisseau/l'élément de verrou (6) en direction de sa position de libération, dans lequel
- le coulisseau/l'élément de verrou (6) ou l'élément de ressort de pression (14) est formé ou pourvu d'une section de retenue/d'un élément de retenue actionnable de préférence manuellement qui peut être amené en prise de retenue avec un élément de blocage/de prise actionnable de préférence manuellement au niveau de ou sur des côtés du couvercle de récipient (2) lorsque la position de fermeture est atteinte afin de retenir le coulisseau/l'élément de verrou (6) en position de fermeture à l'encontre de la force de précontrainte du ressort de rappel (19), position dans laquelle la section de griffe (9) vient en prise sous un bord de bac de récipient et attire le couvercle de récipient (2) contre le bord de bac de récipient suite à la force de ressort de l'au moins un élément de ressort de pression (14) jusqu'à l'actionnement manuel de l'élément de blocage/de prise sur des côtés du couvercle de récipient (2) ou de l'élément de retenue/la section de retenue sur des côtés de l'élément de ressort de pression (14) pour une libération du coulisseau/de l'élément de verrou (6), et le coulisseau/l'élément de verrou (6) revient immédiatement dans sa position de libération en raison du ressort de rappel (19).
